# EUROPÄISCHE PATENTSCHRIFT

(11) **EP 1 794 111 B1**
(45) Veröffentlichungstag und Bekanntmachung des Hinweises auf die Patenterteilung: **16.07.2014**
(21) Anmeldenummer: 05794143.7
(22) Anmeldetag: 23.09.2005
(51) Int. Cl.: C07C 45/28, C07C 49/395

(54) **VERFAHREN ZUR HERSTELLUNG VON CYCLOPENTANON**
METHOD FOR THE PRODUCTION OF CYCLOPENTANONE
PROCEDE DE PREPARATION DE LA CYCLOPENTANONE

(30) Priorität: 23.09.2004 DE 102004046171
(43) Veröffentlichungstag der Anmeldung: 13.06.2007
(73) Patentinhaber: BASF SE, 67056 Ludwigshafen (DE)
(72) Erfinder: TELES, Joaquim, 67166 Otterstadt (DE); RÖSSLER, Beatrice, 67098 Bad Dürkheim (DE); GENGER, Thomas, 67245 Lambsheim (DE); GLASS, Andreas, 67245 Lambsheim (DE)
(86) Internationale Anmeldenummer: PCT/EP2005/010346
(87) Internationale Veröffentlichungsnummer: WO 2006/032532

(56) Entgegenhaltungen:
- GB-A- 649 680
- DATABASE CHEMABS ONLINE CHEMICAL ABSTRACTS SERVICE, COLUMBUS, OHIO, US; database accession no. 138:26062 9. Januar 2003 (2003-01-09), DUBKOV, K.A.; PANOV, G.I. ET AL: "Non-catalytic liquid phase oxidation of alkenes with nitrous oxide. 2. Oxidation of cyclopentene to cyclopentanone" XP002296643 in der Anmeldung erwähnt & REACTION KINETICS AND CATALYSIS LETTERS, Bd. 77, Nr. 1, 2002, Seiten 197-205,
- DATABASE CHEMABS ONLINE CHEMICAL ABSTRACTS SERVICE, COLUMBUS, OHIO, US; database accession number 139:276656 23. Oktober 2003 (2003-10-23), PANOV ET AL: "Method for producing monocyclic ketones C4-7" XP002296644 in der Anmeldung erwähnt & WO 03/078372 A (INSTITUT KATALIZA IMENI G.K. BORESKOVA SIBIRSKOGO; PANOV, GENNADY IVAN) 25. September 2003 (2003-09-25)

## Beschreibung

Die vorliegende Erfindung betrifft ein Verfahren zur Herstellung von Cyclopentanon, ausgehend von einem Gemisch, das Cyclopenten zu höchstens 95 Gew.-% enthält. Neben Cyclopenten kann das Gemisch weitere Lösungsmittel enthalten, wobei beispielsweise weitere Kohlenwasserstoffe als Gemischbestandteile bevorzugt sind. Erfindungsgemäß wird dabei Cyclopentanon durch Umsetzung mit N₂O erhalten, wobei entweder reines N₂O oder ein Gasgemisch, das N₂O enthält, in flüssiger oder überhitischer Form eingesetzt wird.

Cyclopentanon wird großtechnisch derzeit im Wesentlichen ausschließlich durch katalytische Zyklisierung von Adipinsäure bei hoher Temperatur hergestellt. Diese Reaktion liefert zwar eine gute Ausbeute an Cyclopentanon, doch gehen ungefähr 42 % der eingesetzten Adipinsäure in Form von Kohlendioxid und Wasser verloren. Diese niedrige Atomeffizienz belastet unter anderem die Wirtschaftlichkeit des Verfahrens. Zwar ist die Herstellung auch ausgehend von Adipinsäureester möglich, doch ist die Atomeffizienz in diesem Fall noch geringer.

In der Literatur wird als Alternative im Labormaßstab die Oxidation von Cyclopenten mit N₂O angeboten. So offenbart die GB 649,680 die Umsetzung von Alkenen wie beispielsweise Cyclohexen oder Cyclopenten mit N₂O. Allerdings ist in den Beispielen dieser Schrift die Umsetzung von Cyclopenten mit N₂O explizit nicht beschrieben. Andere, in den Beispielen mit N₂O umgesetzte und nicht substituierte Olefine werden entweder als reine Verbindungen oder zusammen mit dem Lösungsmittel Diethylanilin eingesetzt.

Die zur GB 649,680 äquivalente US 2,636,898 offenbart in den Beispielen ebensowenig die Umsetzung von Cyclopenten mit N₂O. Auch in diesen Beispielen werden die nicht substituierten Olefine ausschließlich in reiner Form ohne Zusatz eines Lösungsmittels mit N₂O umgesetzt.

F. S. Bridson-Jones et al. beschreiben in J. Chem. Soc., S. 2999-3008 (1951) die Umsetzung von Olefinen mit N₂O, wobei beispielsweise Cyclohexen zu Cyclohexanon umgesetzt wird. Auch in diesem Fall wird das Cyclohexen als solches ohne Zugabe beispielsweise eines zusätzlichen Lösungsmittels eingesetzt. Ebenso sind beispielsweise die Umsetzung von Ethylen, Acenaphthylen und Methylencyclobutan beschrieben, wobei entweder Cyclohexan oder Decalin als Lösungsmittel eingesetzt wird.

Auch K. A. Dubkov et al., React. Kinet. Catal. Lett., Vol. 77, No. 1, S. 197-205 (2002) beschreiben die Umsetzung von reinem, 99 %igem Cyclopenten mit reinem N₂O (pure, medical grade). Wie bereits in allen anderen zitierten Schriften wird bei der Umsetzung kein Lösungsmittel eingesetzt. Ebenso ist bei der Umsetzung neben Cyclopenten kein weiterer Kohlenwasserstoff anwesend.

In den neueren wissenschaftlichen Artikeln von G. L. Panov et al., "Non-Catalytic Liquid Phase Oxidation of Alkenes with Nitrous Oxide. 1. Oxidation of Cyclohexene to Cyclohexanone", React. Kinet. Catal. Lett. Vol. 76, No. 2 (2002) S. 401-405, und K. A. Dubkov et al., "Non-Catalytic Liquid Phase Oxidation of Alkenes with Nitrous Oxide. 2. Oxidation of Cyclopentene to Cyclopentanone", React. Kinet. Catal. Lett. Vol. 77, No. 1 (2002) S. 197-205 werden ebenfalls Oxidationen von olefinischen Verbindungen mit Distickstoffmonoxid beschrieben. Auch ein wissenschaftlicher Artikel "Liquid Phase Oxidation of Alkenes with Nitrous Oxide to Carbonyl Compounds" von E. V. Starokon et al. in Adv. Synth. Catal. 2004, 346, 268 - 274 beinhaltet eine mechanistische Studie der Oxidation von Alkenen mit Distickstoffmonoxid in flüssiger Phase.

Die Synthese von Carbonylverbindungen aus Alkenen mit Distickstoffmonoxid wird auch in verschiedenen internationalen Patentanmeldungen beschrieben. So offenbart die WO 03/078370 ein Verfahren zur Herstellung von Carbonylverbindungen aus aliphatischen Alkenen mit Distickstoffmonoxid. Die Umsetzung wird bei Temperaturen im Bereich von 20 bis 350°C und Drücken von 1,01325 bis 10132,5 kPa (0,01 bis 100 atm) durchgerührt. Die WO 03/078374 offenbart ein entsprechendes Verfahren zur Herstellung von Cyclohexanon. Gemäß der WO 03/078372 werden cyclische Ketone mit 4 bis 5 C-Atomen hergestellt. Gemäß der WO 03/078375 werden unter diesen Verfahrensbedingungen cyclische Ketone aus cyclischen Alkenen mit 7 bis 20 Atomen hergestellt. WO03/078371 offenbart ein Verfahren zur Herstellung substituierter Ketone aus substituierten Alkenen. WO 04/000777 offenbart ein Verfahren zum Umsetzung von Di- und Polyalkenen mit Distickstoffmonoxid zu den entsprechenden Carbonylverbindungen. Die Reinigung von Distickstoffmonoxid wird in diesen Schriften nicht erwähnt.

Die Verwendung von reinen Edukten, wie in den oben zitierten wissenschaftlichen Arbeiten beschrieben, mag zwar für die Aufklärung von Reaktionsmechanismen von großem Wert sein, ist aber in der Praxis mit einigen Nachteilen verbunden. So ist beispielsweise Cyclopenten in der oben beschriebenen hohen Reinheit nur mit sehr hohem technischen Aufwand zu erhalten. In technischen Prozessen, in denen Cyclopenten in den erforderlichen großtechnischen Mengen anfällt, liegt dieses in Gemischen mit anderen Kohlenwasserstoffen wie beispielsweise Cyclopentan vor. Bedingt durch die ähnlichen Siedepunkte ist die Auftrennung über beispielsweise destillative Methoden mit einem erheblichen Aufwand verbunden.

Eine der der vorliegenden Erfindung zugrunde liegenden Aufgaben war es daher, ein Verfahren bereitzustellen, das es erlaubt, bei der Cyclopentanon-Herstellung als Edukt Cyclopenten, das in einem Gemisch vorliegt, das höchstens 95 Gew.-% Cyclopenten aufweist, einzusetzen, wie es beispielsweise in großtechnischen Verfahren anfällt.

Demgemäß betrifft die vorliegende Erfindung ein Verfahren zur Herstellung von Cyclopentanon, mindestens umfassend folgende Schritte (i) bis (iü):
(i) Bereitstellen eines Cyclopenten enthaltenden Gemisches G(i);
(ii) Bereitstellen von füssigem oder überkritischem N₂O oder eines verflüssigten oder überkritischen Gasgemisches G(ii), enthaltend mindestens 20 Vol.-% N₂O, bezogen auf das Gesamtvolumen des Gemisches G(ii);
(iii) Inkontaktbringen des Gemisches G(i) mit dem flüssigen oder überkritischen N₂O oder mit dem verflüssigten oder überkritischen Gemisch G(ii) unter Erhalt eines Gemisches G(iii), enthaltend Cyclopentanon,
das dadurch gekennzeichnet ist, dass das Gemisch G(i) mindestens 25 Gew.-% und höchstens 95 Gew.-% Cyclopenten, bezogen auf das Gesamtgewicht des Gemisches G(i), enthält, und wobei in Schritt (iii) die Gemische G(i) und G(ii) in einem kontinuierlichen Reaktor in Kontakt gebracht werden bei Temperaturen im Bereich von 200 bis 290°C und Drücken im Bereich von 10 bis 400 bar, wobei die Veiweilzeit des Reaktionsgemisches im kontinuierlichen Reaktor im Bereich von 0,3 bis 2,5 h liegt.

Vorzugsweise wird flüssiges N₂O oder ein verflüssigtes Gemisch G(ii) eingesetzt.

Die DE 103 19 489.4 offenbart ein Verfahren zur Herstellung von Cyclopentanon unter Verwendung von Distickstoffmonoxid als Oxidationsmittel. Die Verwendung von flüssigem oder überkritischem Distickstoffmonoxid wird dort jedoch nicht erwähnt.

Grundsätzlich kann das Gemisch G(i) zusätzlich zu Cyclopenten jede weitere Verbindung enthalten. Geeignet sind unter anderem auch Verbindungen, die bei dem Inkontaktbringen gemäß (iii) ebenfalls mit N₂O reagieren können. Bevorzugt sind hierbei solche Verbindungen, die zwar prinzipiell mit N₂O reagieren können, bei den gemäß (iii) gewählten Reaktionsbedingungen jedoch gegenüber N₂O inert sind. Der Begriff "inert", wie er im Rahmen der vorliegenden Erfindung verwendet wird, bezeichnet Verbindungen, die bei den gemäß (iii) gewählten Reaktionsbedingungen mit N₂O entweder nicht reagieren oder im Vergleich zur Reaktion von Cyclopenten mit N₂O derart eingeschränkt reagieren, dass ihr Umsetzungsprodukt mit N₂O im aus (iii) resultierenden Gemisch zu höchstens 15 Gew.-%, bevorzugt zu höchstens 10 Gew.-% und besonders bevorzugt zu höchstens 5 Gew.-%, jeweils bezogen auf das Gesamtgewicht der aus (iii) resultierenden Mischung, enthalten ist.

Demgemäß betrifft die vorliegende Erfindung auch ein Verfahren, wie oben beschrieben, das dadurch gekennzeichnet ist, dass das Gemisch G(i) zusätzlich zu Cyclopenten mindestens eine beim Inkontaktbringen gemäß (iii) gegenüber N₂O inerte Verbindung enthält.

Als inerte Verbindungen sind im Rahmen der vorliegenden Erfindung Alkane wie beispielsweise Cyclopentan, Hexan, Octan, Decan, Dodecan oder Benzol oder Alkylbenzole wie beispielsweise Toluol, Xylole, Ethylbenzol oder Ether wie beispielsweise Methyl-tert-Butylether, Tetrahydrofuran, Diethylether oder Ester wie beispielsweise Methylacetat, Ethylacetat, Methylbenzoat oder Nitrile wie beispielsweise Acetonitril, Benzonitril oder Alkohole wie beispielsweise Butanol, 2-Ethylhexanol, Ethanol oder Phenole wie beispielsweise Phenol, Kresole oder Amine wie beispielsweise Anilin, Triethylamin, N,N-Dimethylanilin oder Gemische aus zwei oder mehr der genannten Verbindungen oder zwei oder mehr Verbindungen der genannten Klassen geeignet.

Ganz besonders bevorzugt sind solche Verbindungen, die bei den gemäß (iii) gewählten Reaktionsbedingungen mit N₂O nicht reagieren.

Im Rahmen einer diesbezüglich bevorzugten Ausführungsform des erfindungsgemäßen Verfahrens wird als Eduktgemisch G(i) ein Gemisch eingesetzt, das aus Spaltung und Partialhydrierung von Dicyclopentadien in Anwesenheit eines Lösungsmittels gewonnen wird und Cyclopenten enthält, wobei das Lösungsmittel aus den oben genannten inerten Verbindungen ausgewählt wird. Bevorzugt wird hierbei zur Partialhydrierung ein 2:1-Gemisch aus Dicyclopentadien und Toluol eingesetzt. Dieses Verfahren ist beispielsweise in der JP 2000053597 A beschrieben, die diesbezüglich durch Bezugnahme in den Kontext der vorliegenden Anmeldung vollumfänglich einbezogen wird. Gemäß der JP 200053597 wird Cyclopentadien durch Thermolyse von Dicyclopentadien in Anwesenheit eines aromatischen Kohlenwasserstoffs, bevorzugt Toluol, erhalten, wobei die Umsetzungsrate 98% beträgt. Das erhaltene Gas wird in ein Edelstahkeaktionsrohr geleitet, das mit Palladium/Aluminiumoxid-Katalysator gefüllt ist. Das Gas am Auslass des Reaktionsrohres wird mit einem Kühler kondensiert.

Gemäß einer weiteren bevorzugten Ausführungsform des erfindungsgemäßen Verfahrens besteht das Gemisch G(i) zu mindestens 99 Gew.-%, bezogen auf das Gesamtgewicht des Gemisches G(i), aus Kohlenwasserstoffen. Neben den Kohlenwasserstoffen kann demgemäß das Gemisch G(i) noch zu höchstens 1 Gew.-% mindestens eine weitere Verbindung enthalten, wobei unter anderem mindestens eine der oben genannten, von Kohlenwasserstoffen verschiedene, inerten bevorzugten Verbindungen zu höchstens 1 Gew.-% enthalten sein kann. Auch andere Verbindungen können zu höchstens 1 Gew.-% unter der Maßgabe enthalten sein, dass sie die Umsetzung von Cyclopenten gemäß (iii) nicht stören.

Der Begriff "Kohlenwasserstoffgemisch", wie er im Rahmen der vorliegenden Erfindung verwendet wird, bezeichnet ein Gemisch aus Verbindungen, von denen jede ein nicht substituierter Kohlenwasserstoff ist und daher nur aus den Atomen C und H besteht. Die im Rahmen der vorliegenden Erfindung eingesetzten Kohlenwasserstoffgemische enthalten dabei zu höchstens 1 Gew.-%, bezogen auf das Gesamtgewicht des jeweiligen Gemisches G(i), weitere Verbindungen. Weiter bevorzugt enthält das Gemisch zu höchstens 0,5 Gew.-%, weiter bevorzugt zu höchstens 0,1 Gew.-%, weiter bevorzugt zu höchstens 0,01 Gew.-% und ganz besonders bevorzugt zu höchstens 0,001 Gew.-% weitere Verbindungen. Insbesondere bevorzugt sind Gemische G(i), die im Rahmen der Messgenauigkeit der jeweils eingesetzten Untersuchungsmethoden keine weiteren Verbindungen enthalten.

Gemäß einer bevorzugten Ausführungsform ist das Gemisch G(i) bei den gemäß (iii) gewählten Reaktionsbedingungen flüssig oder überkritisch. Unter anderem bevorzugt sind hierbei Gemische G(i), die bei Umgebungstemperatur und Umgebungsdruck flüssig sind. Hierbei sind etwa Gemische zu nennen, von denen jede enthaltene Verbindung bei Umgebungstemperatur und Umgebungsdruck flüssig ist. Ebenso sind Gemische denkbar, die bei Umgebungstemperatur und Umgebungsdruck flüssig sind und dabei mindestens eine Verbindung enthalten, die beispielsweise bei Umgebungstemperatur und Umgebungsdruck fest oder gasförmig ist, bei Umgebungstemperatur und Umgebungsdruck in dem Gemisch G(i) jedoch gelöst vorliegt.

Im Rahmen einer ebenfalls bevorzugten Ausführungsform des erfindungsgemäßen Verfahrens wird ein Gemisch G(i) eingesetzt, das zu mindestens 99 Gew.-% aus C₅-Kohlenwasserstoffen und Kohlenwasserstoffen mit mehr als 5 Kohlenstoffatomen besteht. Neben Cyclopenten können demgemäß mindestens ein weiterer C₅-Kohlenwasserstoff oder mindestens ein Kohlenwasserstoff mit mehr als 5 Kohlenstoffatomen oder ein Gemisch aus mindestens einem weiteren C₅-Kohlenwasserstoff und mindestens einem Kohlenwasserstoff mit mehr als 5 Kohlenstoffatomen in G(i) enthalten sein.

Demgemäß beschreibt die vorliegende Erfindung auch ein Verfahren, wie oben beschrieben, das dadurch gekennzeichnet ist, dass das Gemisch G(i) mindestens 99 Gew.-% C₅-Kohlenwasserstoffe und Kohlenwasserstoffe mit mehr als 5 Kohlenstoffatomen enthält.

Als unter anderem besonders bevorzugte Kohlenwasserstoffe mit mehr als 5 Kohlenstoffatomen werden die entsprechenden, bereits oben im Rahmen der inerten Verbindungen genannten Kohlenwasserstoffe eingesetzt.

Wie bereits oben erwähnt, werden als Eduktgemische G(i) bevorzugt solche Gemische eingesetzt, wie sie in großtechnischen Verfahren anfallen. Im Rahmen der vorliegenden Erfindung sind hierbei Gemische bevorzugt, die zu mindestens 95 Gew.-%, weiter bevorzugt zu mindestens 97 Gew.-% und besonders bevorzugt zu mindestens 99 Gew.-% aus C₅-, C₆- und C₇-Kohlenwasserstoffen bestehen.

Demgemäß betrifft die vorliegende Erfindung auch ein Verfahren, wie oben beschrieben, das dadurch gekennzeichnet ist, dass das Gemisch G(i) zu mindestens 99 Gew.-% C₅- und C₆- oder C₅- und C₇- oder C₅- und C₆- und C₇-Kohlenwasserstoffe enthält.

Im Rahmen der vorliegenden Erfindung kann dabei das Gemisch G(i) neben Cyclopenten entweder mindestens einen weiteren C₅-Kohlenwasserstoff oder mindestens einen C₆-Kohlenwasserstoff oder mindestens einen C₇-Kohlenwasserstoff oder ein Gemisch aus mindestens einem weiteren C₅-Kohlenwasserstoff und mindestens einem C₆-Kohlenwasserstoff oder ein Gemisch aus mindestens einem weiteren C₅-Kohlenwasserstoff und mindestens einem C₇-Kohlenwasserstoff oder ein Gemisch aus mindestens einem weiteren C₅-Kohlenwasserstoff und mindestens einem C₆-Kohlenwasserstoff und mindestens einem C₇-Kohlenwasserstoff enthalten.

Im Rahmen einer bevorzugten Ausführungsform des erfindungsgemäßen Verfahrens wird als Eduktgemisch G(i) ein Kohlenwasserstoffgemisch eingesetzt, das aus einem Steamcracker oder einer Raffinerie gewonnen wird und Cyclopenten enthält. In diesem Zusammenhang sind beispielsweise C₅-Schnitte aus Steamcracker-Anlagen bevorzugt, die im Wesentlichen nur C₅- und C₆-Kohlenwasserstoffe enthalten. Kohlenwasserstoffe mit mehr als 6 Kohlenstoffatomen sind in den großtechnisch anfallenden C₅-Schnitten nicht enthalten, die neben Cyclopenten beispielsweise 2-Buten, Isopentan, 1-Penten, 2-Methylbuten-1, trans-2-Penten, n-Pentan, cis-2-Penten, 2-Methylbuten-2, Cyclopentan, 2,2-Dimethylbutan, 2-Methylpentan, 3-Methylpentan, n-Hexan und Benzol umfassen. Im Allgemeinen enthält ein C₅-Schnitt aus einer Steamcracker-Anlage Cyclopenten im Bereich von 5 bis 60 Gew.-% und bevorzugt im Bereich von 15 bis 50 Gew.-%.

Daher beschreibt die vorliegende Erfindung auch ein Verfahren, wie oben beschrieben, das dadurch gekennzeichnet ist, dass das Gemisch G(i) zu mindestens 99 Gew.-% ein Gemisch aus C₅- und C₆-Kohlenwasserstoffen enthält.

Erfindungsgemäß kann dieses Gemisch aus im wesentlichen C₅- und C₆-Kohlenwasserstoffen, das bevorzugt als C₅-Schnitt aus einer Steamcracker-Anlage gewonnen wird, als solches eingesetzt werden. Bevorzugt wird das Gemisch aus im wesentlichen C₅- und C₆-Kohlenwasserstoffen vor der erfindungsgemäßen Umsetzung gemäß Schritt (iii) einer Reinigung unterzogen, bei der wiederum bevorzugt im Vergleich zu Cyclopenten leichter siedende Verbindungen abgetrennt werden. Während hierbei sämtliche denkbaren Methoden einsetzbar sind, ist die destillative Auftrennung des Gemisches bevorzugt.

Im Rahmen der vorliegenden Erfindung werden hierbei bevorzugt Gemische G(i) erhalten, die zu höchstens 10 Gew.-% C₅- und/oder C₆-Kohlenwasserstoffe enthalten, die leichter sieden als Cyclopenten. Sollte im zu reinigenden Gemisch G(i) gegebenenfalls zusätzlich mindestens ein C₄-Kohlenwasserstoff enthalten sein, so werden durch die bevorzugt eingesetzte Destillation bevorzugt Gemische G(i) erhalten, die zu höchstens 10 Gew.-% C₄- und/oder C₅- und/oder C₆-Kohlenwasserstoffe enthalten, die leichter sieden als Cyclopenten. Im Rahmen der vorliegenden Erfindung werden hierbei besonders bevorzugt Gemische G(i) erhalten, die zu höchstens 5 Gew.-%, weiter bevorzugt zu höchstens 3 Gew.-% und besonders bevorzugt zu höchstens 2 Gew.-% C₅- und/oder C₆-Kohlenwasserstoffe enthalten, die leichter sieden als Cyclopenten. Sollte im zu reinigenden Gemisch G(i) gegebenenfalls zusätzlich mindestens ein C₄-Kohlenwasserstoff enthalten sein, so werden durch die bevorzugt eingesetzte Destillation bevorzugt Gemische G(i) erhalten, die zu höchstens 5 Gew.-%, weiter bevorzugt zu höchstens 3 Gew.-% und besonders bevorzugt zu höchstens 2 Gew.-% C₄- und/oder C₅- und/oder C₆-Kohlenwasserstoffe enthalten, die leichter sieden als Cyclopenten.

Demgemäß beschreibt die vorliegende Erfindung auch ein Verfahren, wie oben beschrieben, das dadurch gekennzeichnet ist, dass das Gemisch G(i) mindestens 99 Gew.-%, bezogen auf das Gesamtgewicht des Gemisches G(i), an C₅- und C₆-Kohlenwasserstoffen und höchstens 2 Gew.-%, bezogen auf das Gesamtgewicht des Gemisches G(i), an im Vergleich zu Cyclopenten leichter siedenden Kohlenwasserstoffen enthält.

Im Rahmen einer ebenfalls bevorzugten Ausführungsform des erfindungsgemäßen Verfahrens wird ein Gemisch G(i) eingesetzt, das zu mindestens 99 Gew.-% aus C₅- und C₇-Kohlenwasserstoffen besteht. Neben Cyclopenten können demgemäß mindestens ein weiterer C₅-Kohlenwasserstoff oder mindestens ein C₇-Kohlenwasserstoff oder ein Gemisch aus mindestens einem weiteren C₅-Kohlenwasserstoff und mindestens einem C₇-Kohlenwasserstoff in G(i) enthalten sein.

Demgemäß beschreibt die vorliegende Erfindung auch ein Verfahren, wie oben beschrieben, das dadurch gekennzeichnet ist, dass das Gemisch G(i) mindestens 99 Gew.-% C₅- und C₇-Kohlenwasserstoffe enthält.

Als C₇-Kohlenwasserstoff sei beispielsweise besonders bevorzugt Toluol genannt.

Im Rahmen einer diesbezüglich bevorzugten Ausführungsform des erfindungsgemäßen Verfahrens wird als Eduktgemisch G(i) ein Kohlenwasserstoffgemisch eingesetzt, das aus Spaltung und Partialhydrierung von Dicyclopentadien in Anwesenheit von Toluol als Lösungsmittel gewonnen wird und Cyclopenten enthält. Bevorzugt wird hierbei zur Partialhydrierung ein 2:1-Gemisch aus Dicyclopentadien und Toluol eingesetzt. Dieses Verfahren ist beispielsweise in der JP 2000053597 A beschrieben.

Die derart erhaltenen Gemische enthalten im allgemeinen Cyclopenten in einem Bereich von 25 bis 75 Gew.-%, bevorzugt in einem Bereich von 35 bis 65 Gew.-% und besonders bevorzugt in einem Bereich von 40 bis 60 Gew.-%. Neben Cyclopenten enthalten die Reaktionsgemische hauptsächlich Cyclopentan und Toluol. Im allgemeinen besteht das aus Spaltung und Partialhydrierung eines Gemisches aus Dicyclopentadien und Toluol gewonnene Gemisch, das als Gemisch G(i) im Rahmen des erfindungsgemäßen Verfahrens eingesetzt werden kann, zu mindestens 99 Gew.-% aus Cyclopenten, Toluol und Cyclopentan.

Das gemäß dieser bevorzugten Ausführungsform erhaltene Gemisch, das zu mindestens 99 Gew.-% aus Cyclopenten, Toluol und Cyclopentan besteht, kann als solches eingesetzt werden.

Gemäß einer weiter bevorzugten Ausführungsform wird das aus der Spaltung und Partialhydrierung eines Gemisches aus Dicyclopentadien und Toluol gewonnene Gemisch vor dem Einsatz als Gemisch G(i) im erfindungsgemäßen Verfahren mindestens einer destillativen Trennung unterzogen, in dem ein Leichtsiedergemisch erhalten wird, das Cyclopenten im allgemeinen im Bereich von 60 bis 95 Gew.-%, bevorzugt im Bereich von 70 bis 90 Gew.-% und besonders bevorzugt im Bereich von 75 bis 85 Gew.-% enthält. Weiter enthält dieses Leichtsiedergemisch Toluol im allgemeinen im Bereich von höchstens 20 Gew.-%, bevorzugt von höchstens 10 Gew.-% und besonders bevorzugt von höchstens 5 Gew.-%, und Cyclopentan im allgemeinen im Bereich von 5 bis 25 Gew.-%, bevorzugt im Bereich von 7 bis 22 Gew.-% und besonders bevorzugt im Bereich von 10 bis 20 Gew.-%. Dieses Leichtsiedergemisch wird dann im erfindungsgemäßen Verfahren als Gemisch G(i) eingesetzt.

Weiter bevorzugt enthält das im Rahmen des erfindungsgemäßen Verfahrens eingesetzte Gemisch G(i) Cyclopenten in einem Bereich von 30 bis 90 Gew.-%, besonders bevorzugt in einem Bereich von 40 bis 90 Gew.-%, weiter besonders bevorzugt in einem Bereich von 45 bis 90 Gew.-% und insbesondere bevorzugt in einem Bereich von 50 bis 85 Gew.-%, jeweils bezogen auf das Gesamtgewicht des Gemisches G(i).

Erfindungsgemäß wird zur Umsetzung gemäß Schritt (iii) ein zu mindestens 20 Vol.-% N₂O enthaltendes Gasgemisch G(ii) eingesetzt, wobei im allgemeinen auch reines N₂O eingesetzt werden kann, wobei das Gasgemisch G(ii) oder das N₂O in flüssiger oder überkritischer Form eingesetzt wird, vorzugsweise in flüssiger Form. Dabei werden Druck und Temperatur vorzugsweise derart gewählt, dass das Gasgemisch in flüssiger oder überkritischer Form vorliegt, besonders bevorzugt in flüssiger Form. Dabei ist es erfindungsgemäß auch möglich, dass das Gasgemisch G(ü) oder das N₂O in einem Lösungsmittel absorbiert werden.

Der Begriff "Gasgemisch", wie er im Rahmen der vorliegenden Erfindung verwendet wird, bezeichnet ein Gemisch aus zwei oder mehr Verbindungen, die sich bei Umgebungsdruck und Umgebungstemperatur im gasförmigen Zustand befinden. Bei veränderter Temperatur oder verändertem Druck kann das Gasgemisch auch in einem anderen Aggregatzustand vorliegen, beispielsweise flüssig, und wird im Rahmen der vorliegenden Erfindung weiter als Gasgemisch bezeichnet.

Erfindungsgemäß wird dieses Gasgemisch verflüssigt und dann in flüssiger Form eingesetzt. Dabei kann Distickstoffmonoxid oder das Gasgemisch enthaltend Distickstoffmonoxid mit allen dem Fachmann bekannten Verfahren verflüssigt werden, insbesondere durch geeignete Wahl des Drucks und der Temperatur.

Gemäß einer bevorzugten Ausführungsform wird ein mindestens 20 Vol.-% N₂O enthaltendes Gasgemisch G(ii) eingesetzt, wobei wiederum bevorzugt Gemische G(ii) mit einem N₂O-Gehalt im Bereich von 20 bis 97 Vol.-%, weiter bevorzugt im Bereich von 30 bis 95 Vol.-%, weiter bevorzugt im Bereich von 40 bis 94 Vol.-% und insbesondere bevorzugt im Bereich von 50 bis 93 Vol.-% eingesetzt werden.

Im Rahmen der vorliegenden Erfindung wird die Zusammensetzung der Gasgemische oder der verflüssigten Gasgemische in Vol.-% angegeben. Dabei beziehen sich die Angaben auf die Zusammensetzung der Gasgemische bei Umgebungsdruck und Umgebungstemperatur.

Demgemäß betrifft die vorliegende Erfindung auch ein Verfahren, wie oben beschrieben, das dadurch gekennzeichnet ist, dass das Gemisch G(ii) höchstens 93 Vol.-% N₂O enthält.

Wird ein Gasgemisch G(ii) eingesetzt, so kann dieses neben N₂O noch mindestens ein weiteres Gas enthalten. Hierbei sind im Wesentlichen sämtliche Gase denkbar, solange gewährleistet ist, dass die Umsetzung von Cyclopenten mit N₂O gemäß Schritt (iii) möglich ist. Insbesondere sind demzufolge Gemische G(ii) bevorzugt, die neben N₂O mindestens ein Inertgas enthalten. Der Begriff "Inertgas", wie er im Rahmen der vorliegenden Erfindung verwendet wird, bezeichnet ein Gas, das sich hinsichtlich der Umsetzung von N₂O mit Cyclopenten inert verhält. Als Inertgase sind beispielsweise Stickstoff, Kohlendioxid, Kohlenmonoxid, Argon, Methan, Ethan und Propan zu nennen. Ebenso können im Gemisch G(ii) auch Gase enthalten sein, die sich bei der Umsetzung von N₂O mit Cyclopenten nicht als Inertgase verhalten. Als solche Gase sind unter anderem NOₓ oder beispielsweise Sauerstoff zu nennen. Der Begriff "NOₓ", wie er im Rahmen der vorliegenden Erfindung verstanden wird, bezeichnet sämtliche Verbindungen NₐO_{b} außer N₂O, wobei a 1 oder 2 ist und b eine Zahl von 1 bis 6. Statt dem Begriff "NOₓ" wird im Rahmen der vorliegenden Erfindung auch der Begriff "Stickoxide" verwendet. In einem solchen Fall ist es bevorzugt, solche Gemische G(ii) einzusetzen, deren Gehalt an diesen Gasen höchstens 0,5 Vol.-%, bezogen auf das Gesamtgewicht des Gemisches G(ii), beträgt.

Demgemäß betrifft die vorliegende Erfindung auch ein Verfahren, wie oben beschrieben, das dadurch gekennzeichnet ist, daß das Gemisch G(ü) höchstens 0,5 Vol-% Sauerstoff oder höchstens 0,5 Vol-% Stickoxide oder höchstens sowohl 0,5 Vol-% Sauerstoff als auch 0,5 Vol-% Stickoxide, jeweils bezogen auf das Gesamtvolumen des Gemisches G(ii), enthält. Ein Wert von beispielsweise 0,5 Vol.-% bezeichnet hierbei einen Gesamtgehalt aller möglichen Stickoxide außer N₂O von 0,5 Vol.-%.

Grundsätzlich kann die Zusammensetzung der Gemische im Rahmen der vorliegenden Erfindung auf jede dem Fachmann bekannte Weise bestimmt werden. Die Zusammensetzung der Gasgemische G(ii) wird im Rahmen der vorliegenden Erfindung gaschromatographisch bestimmt. Sie kann jedoch auch mittels UV-Spektroskopie, IR-Spektroskopie oder nasschemisch bestimmt werden.

Erfindungsgemäß wird das Gasgemisch G(ii) in flüssiger oder überkritischer Form eingesetzt, vorzugsweise in flüssiger Form. Dabei ist es erfindungsgemäß möglich, dass das Gasgemisch (ü) vor der Verflüssigung einer Behandlung unterzogen wird, um die Konzentration von inerten und störenden Verbindungen im Gasgemisch G(ii) zu reduzieren.

Diese Behandlung kann erfindungsgemäß beispielsweise eine Absorption des Gasgemisches in einem geeigneten Lösungsmittel und anschließende Desorption umfassen, um inerte Verbindungen aus dem Gasgemisch zu entfernen. Ein geeignetes Lösungsmittel ist beispielsweise Wasser, wie in der DT 20 40 219 beschrieben.

Erfindungsgemäß kann die Behandlung des Gasgemisches auch einen Reinigungsschritt zur Abtrennung von NOₓ aus dem Gasgemisch umfassen. Derartige Verfahren zur Abtrennung von NOₓ sind grundsätzlich aus dem Stand der Technik bekannt. Erfindungsgemäß können alle dem Fachmann bekannten Verfahren zur Abtrennung von NOₓ eingesetzt werden.

Insbesondere ist es im Rahmen der vorliegenden Erfindung möglich, Gasgemische G(ii) einzusetzen, die aus großtechnischen Verfahren erhalten werden. Sollten demgemäß diese Gemische G(ii) mehr als 0,5 Vol.-% Sauerstoff und/oder Stickoxide enthalten, so können diese im Allgemeinen im erfindungsgemäßen Verfahren eingesetzt werden. Bevorzugt werden diese Gemische G(ii), wie auch solche Gemische G(ii) ähnlicher Zusammensetzung, die nicht aus großtechnischen Verfahren erhalten werden, vor dem Einsatz im erfindungsgemäßen Verfahren mindestens einem Reinigungsschritt unterworfen, in dem der Gehalt an Sauerstoff und/oder Stickoxiden auf höchstens 0,5 Vol.-% eingestellt wird.

Gemäß einer bevorzugten Ausführungsform der vorliegenden Erfindung wird als Gasgemisch G(ii) mindestens ein N₂O enthaltendes Abgas eines chemischen Verfahrens eingesetzt.

Gemäß einer bevorzugten Ausführungsform der vorliegenden Erfindung ist die Distickstoffmonoxidquelle mindestens ein Distickstoffmonoxid enthaltendes Abgas eines chemischen Verfahrens. Im Rahmen der vorliegenden Erfindung sind auch Ausführungsformen umfasst, bei denen mindestens zwei Stickstoffmonoxid enthaltende Abgase einer einzigen Anlage als Distickstoffmonoxidquelle dienen. Ebenso sind Ausführungsformen umfasst, bei denen mindestens ein Distickstoffmonoxid enthaltendes Abgas einer Anlage und mindestens ein weiteres Distickstoffmonoxid enthaltendes Abgas mindestens einer weiteren Anlage als Distickstoffmonoxidquelle dienen.

Demgemäß betrifft die vorliegende Erfindung auch ein wie oben beschriebenes Verfahren, das dadurch gekennzeichnet ist, dass als Distickstoffmonoxidquelle mindestens ein Distickstoffmonoxid enthaltendes Abgas mindestens eines industriellen Verfahrens dient.

Ebenso kann im Rahmen des erfindungsgemäßen Verfahrens Distickstoffmonoxid zum Einsatz im Verfahren gezielt hergestellt werden. Bevorzugt wird dabei unter anderem die Herstellung über die thermische Zersetzung von NH₄NO₃, wie dies beispielsweise in der US 3,656,899 beschrieben ist, deren diesbezüglicher Inhalt durch Bezugnahme in den Kontext der vorliegenden Anmeldung vollumfänglich aufgenommen wird. Ebenfalls bevorzugt wird weiter die Herstellung über die katalytische Oxidation von Ammoniak, wie dies beispielsweise in der US 5,849,257 oder in der WO 98/25698 beschrieben ist.

Der Begriff "Distickstoffmonoxidquelle" bezeichnet im Rahmen der vorliegende Erfindung sowohl Ausführungsformen, in denen das genannte Abgas in unmodifizierter Form in der erfindungsgemäßen Umsetzung von Cyclopenten eingesetzt wird, als auch Ausführungsformen, in denen mindestens eines der genannten Abgase einer Modifikation unterworfen wird.

Der Begriff "Modifikation", wie er in diesem Zusammenhang im Rahmen der vorliegenden Erfindung verwendet wird, bezeichnet jedes geeignete Verfahren, mit dem die chemische Zusammensetzung eines Abgases verändert wird. Demgemäß umfasst der Begriff "Modifikation" unter anderem Ausführungsformen, in denen ein Distickstoffmonoxid enthaltendes Abgas bezüglich des Distickstoffmonoxidgehaltes gemäß mindestens eines geeigneten Verfahrens aufkonzentriert wird. Solche Verfahren sind beispielsweise in der DE-OS 27 32 267, der EP 1 076 217 A2 oder der WO 00/73202 A1 beschrieben.

Erfindungsgemäß ist es dabei bevorzugt, dass das Abgas einer Behandlung umfassend eine Absorption in einem geeigneten Lösungsmittel und anschließende Desorption unterworfen wird, um inerte Verbindungen zu entfernen. Ein geeignetes Lösungsmittel ist beispielsweise Wasser, wie in der DT 20 40 219 beschrieben.

Gemäß einer beispielsweise bevorzugten Ausführungsform des erfindungsgemäßen Verfahrens ist es zur Aufkonzentrierung möglich, das oben genannte, Distickstoffmonoxid enthaltende Abgas mindestens einer Adsorptionskolonne zuzuführen und das Distickstoffmonoxid in mindestens einem organischen Lösungsmittel einzulösen. Als Lösungsmittel ist hierfür beispielsweise Cyclopenten geeignet. Diese erfindungsgemäße Verfahrensvariante bietet den Vorteil, dass die resultierende Lösung von Distickstoffmonoxid in Cyclopenten ohne weitere Aufarbeitung der erfindungsgemäßen Umsetzung zugeführt werden kann. Diese Lösung von Distickstoffmonoxid in Cyclopenten kann Distickstoffmonoxid in sämtlichen denkbaren Konzentrationen bis hin zur Sättigung enthalten. Gemäß anderer Ausführungsformen kann mindestens ein weiteres Lösungsmittel oder ein Gemisch aus Cyclopenten und mindestens einem weiteren Lösungsmittel zur Adsorption verwendet werden. Solche weiteren Lösungsmittel sind beispielsweise alle geeigneten gängigen organischen Lösungsmittel. Bevorzugte Lösungsmittel sind unter anderem N-Methylpyrrolidon, Dimethylformamid, Dimethylsulfoxid, Propylencarbonat, Sulfolan oder N,N-Dimethylacetamid. Wird mindestens ein weiteres Lösungsmittel oder ein Gemisch aus Cyclopenten und mindestens einem weiteren Lösungsmittel eingesetzt, so wird gemäß einer weiter bevorzugten Ausführungsform aus der mit Distickstoffmonoxid angereicherten Lösung in mindestens einem geeigneten Desorptionsschritt das Distickstoffmonoxid zumindest teilweise, bevorzugt im wesentlichen vollständig gewonnen und der erfindungsgemäßen Umsetzung zugeführt.

Gemäß einer weiteren Ausführungsform kann die chemische Zusammensetzung eines Abgases auch durch Zusatz von reinem Distickstoffmonoxid zu dem Abgas verändert werden.

Gemäß einer weiter bevorzugten Ausführungsform der vorliegenden Erfindung stammt das mindestens eine Distickstoffmonoxid enthaltende Abgas aus einer Adipinsäureanlage, einer Dodecandisäureanlage, einer Hydroxylaminanlage und/oder einer Salpetersäureanlage, wobei letztere wiederum bevorzugt mit mindestens einem Abgas einer Adipinsäureanlage, einer Dodecandisäureanlage oder einer Hydroxylaminanlage betrieben wird.

Gemäß einer ganz besonders bevorzugten Ausführungsform wird als Gasgemisch G(ii) der Abgasstrom einer Adipinsäureanlage eingesetzt, bei der durch Oxidation von CycIohexanol/Cyclohexanon-Gemischen mit Salpetersäure pro Mol gebildeter Adipinsäure im Allgemeinen 0,8 bis 1,0 mol N₂O gebildet werden. Wie beispielsweise in A. Uriate et al., Stud. Surf. Sci. Catal. 130, S. 743-748 (2000) beschrieben, enthalten die Abgase von Adipinsäureanlagen jedoch in unterschiedlichen Konzentrationen noch weitere Bestandteile wie unter anderem Stickstoff, Sauerstoff, Kohlendioxid, Kohlenmonoxid, Stickoxide, Wasser und flüchtige organische Verbindungen.

Wie bereits oben beschrieben, kann ein solcher Abgasstrom einer Adipinsäureanlage direkt in dem erfindungsgemäßen Verfahren eingesetzt werden. Bevorzugt wird der Abgasstrom gereinigt. Dazu sind beispielsweise sämtliche Verfahren denkbar, die es ermöglichen, den Gehalt des Abgasstroms an Sauerstoff und/oder Stickoxiden auf jeweils höchstens 0,5 Vol.-% einzustellen. In der oben zitierten Schrift von A. Uriate et al. werden verschiedene Möglichkeiten offenbart, wie ein solcher Abgasstrom zur Verwendung bei der katalytischen Benzolhydroxylierung gereinigt werden kann. Es werden Absorptionsverfahren wie beispielsweise Druckwechselabsorption, Membran-trennverfahren, Tieftemperaturdestillation oder eine Kombination aus selektiver katalytischer Reduktion mit Ammoniak, gefolgt von der katalytischen Entfernung von Sauerstoff beschrieben. Sämtliche dieser Reinigungsmethoden sind auch anwendbar, um den Abgasstrom einer Adipinsäureanlage, der im erfindungsgemäßen Verfahren eingesetzt wird, zu reinigen.

Besonders bevorzugt wird im Rahmen der vorliegenden Erfindung der Abgasstrom einer Adipinsäureanlage im Falle, dass dieser Sauerstoff und/oder Stickoxide zu jeweils mehr als 0,5 Vol.-% enthält, aufgereinigt. Bevorzugt erfolgt diese Aufreinigung destillativ.

Gemäß einer ebenfalls bevorzugten Ausführungsform wird als Gasgemisch G(ii) der Abgasstrom einer Dodecandisäureanlage eingesetzt. Bei dieser Dodecandisäureanlage handelt es sich verglichen mit der Adipinsäureanlage um den im Wesentlichen identischen Anlagentyp.

Eine beispielsweise typische Zusammensetzung eines Abgases einer Dodecandisäureanlage oder einer Adipinsäureanlage ist in der folgenden Tabelle wiedergegeben:

| Komponente | Konzentrationen / Gew.-% |
|---|---|
| NO_{X} | 19 - 25 |
| N₂O | 20 - 28 |
| N₂ | 30 - 40 |
| O₂ | 7 - 10 |
| CO₂ | 2 - 3 |
| H₂O | ~7 |

Der Abgasstrom einer Dodecandisäureanlage kann direkt in dem erfindungsgemäßen Verfahren eingesetzt werden. Bevorzugt wird der Abgasstrom der Dodecandisäureanlage vor dem Einsatz im erfindungsgemäßen Verfahren gereinigt. Dabei wird beispielsweise vorteilhaft der Gehalt des Abgasstroms an Sauerstoff und/oder Stickoxiden auf Gehalte im Bereich von jeweils höchstens 0,5 Vol.-%, also 0 bis 0,5 Vol.-% eingestellt. In der oben zitierten Schrift von A. K. Uriarte et al. werden verschiedene Möglichkeiten offenbart, wie ein solcher Abgasstrom zur Verwendung bei der katalytischen Benzolhydroxylierung gereinigt werden kann. Es werden Absorptionsverfahren wie beispielsweise Druckwechselabsorption, Membran-Trennverfahren, Tieftemperaturdestillation oder eine Kombination aus selektiver katalytischer Reduktion mit Ammoniak, gefolgt von der katalytischen Entfernung von Sauerstoff beschrieben. Sämtliche dieser Reinigungsmethoden sind auch anwendbar, um den Distickstoffmonoxid enthaltenden Abgasstrom einer industriellen Anlage wie beispielsweise einer Dodecandisäureanlage oder einer Adipinsäureanlage oder einer Salpetersäureanlage zu reinigen. Ganz besonders bevorzugt sind die destillative Reinigung und damit die destillative Aufkonzentrierung des Abgasstroms einer Dodecandisäureanlage oder einer Adipinsäureanlage oder einer Salpetersäureanlage.

Besonders bevorzugt wird im Rahmen der vorliegenden Erfindung der Abgasstrom einer Dodecandisäureanlage im Falle, dass dieser Sauerstoff und/oder Stickoxide zu jeweils mehr als 0,5 Vol.-% enthält, aufgereinigt.

Gemäß einer ebenfalls bevorzugten Ausführungsform wird als Gasgemisch G(ii) der Abgasstrom einer Salpetersäureanlage eingesetzt, die ganz oder teilweise mit Distickstoffmonoxid und Stickoxide enthaltenden Abgasen aus anderen Verfahren gespeist wird. In derartigen Salpetersäureanlagen werden Stickoxide adsorbiert und zum größten Teil zu Salpetersäure umgesetzt, während Distickstoffmonoxid nicht umgesetzt wird. Beispielsweise kann eine derartige Salpetersäureanlage durch Stickoxide, die durch gezielte Verbrennung von Ammoniak hergestellt werden, und durch Abgase einer Adipinsäureanlage und/oder durch Abgase einer Dodecandisäureanlage gespeist werden. Ebenso ist es möglich, eine derartige Salpetersäureanlage allein durch Abgase einer Adipinsäureanlage und/oder durch Abgase einer Dodecandisäureanlage zu speisen.

Die Abgase von derartigen Salpetersäureanlagen enthalten grundsätzlich in unterschiedlichen Konzentrationen noch weitere Bestandteile wie unter anderem Stickstoff, Sauerstoff, Kohlendioxid, Kohlenmonoxid, Stickoxide, Wasser und flüchtige organische Verbindungen.

Eine beispielsweise typische Zusammensetzung eines Abgases einer derartigen Salpetersäureanlage ist in der folgenden Tabelle wiedergegeben:

| Komponente | Konzentrationen / Gew.-% |
|---|---|
| NO_{X} | < 0,1 |
| N₂O | 8 - 36 |
| N₂ | 57 - 86 |
| O₂ | 3 - 9 |
| CO₂ | 1 - 4 |
| H₂O | ~0,6 |

Der Abgasstrom einer Salpetersäureanlage kann direkt in dem erfindungsgemäßen Verfahren eingesetzt werden. Bevorzugt wird der Abgasstrom der Salpetersäureanlage vor dem Einsatz im erfindungsgemäßen Verfahren gereinigt. Dabei wird beispielsweise vorteilhaft der Gehalt des Abgasstroms an Sauerstoff und/oder Stickoxiden auf Gehalte im Bereich von höchstens 0,5 Vol.-%, also von jeweils 0 bis 0,5 Vol.-% eingestellt. Geeignete Verfahren, mit denen diese Werte einstellbar sind, sind obenstehend im Rahmen der Adipinsäureanlage und Dodecandisäureanlage beschrieben. Ganz besonders bevorzugt sind auch im Rahmen der Abgase der Salpetersäureanlage die destillative Reinigung und damit die destillative Aufkonzentrierung.

Besonders bevorzugt wird im Rahmen der vorliegenden Erfindung der Abgasstrom einer Salpetersäureanlage im Falle, dass dieser Sauerstoff und/oder Stickoxide zu jeweils mehr als 0,5 Vol.-% enthält, aufgereinigt.

Gemäß einer ebenfalls bevorzugten Ausuhrungsform des erfindungsgemäßen Verfahrens wird als Gasgemisch G(ii) der Abgasstrom einer Hydroxylaminanlage eingesetzt, bei der beispielsweise zunächst Ammoniak mit Luft oder Sauerstoff zu NO oxidiert wird, wobei kleine Mengen an Distickstoftmonoxid als Nebenprodukt gebildet werden. Das NO wird anschließend mit Wasserstoff zu Hydroxylamin hydriert. Nachdem Distickstoffmonoxid unter den Hydrierbedingungen inert ist, reichert es sich im Wasserstoffkreis an. In bevorzugten Verfahrensführungen enthält der Purge-Strom einer Hydroxylaminanlage Distickstofmonoxid im Bereich von 9 bis 13 VoL-% in Wasserstoff. Bevorzugt wird dieser Strom vor dem Einsatz im erfindungsgemäßen Verfahren bezüglich des Distickstoffmonoxidgehaltes, geeignet aufkonzentriert.

Demgemäß beschreibt die vorliegende Erfindung auch ein wie oben beschriebenes Verfahren, das dadurch gekennzeichnet ist, dass als Gasgemisch G(ii) das Abgas einer Adipinsäureanlage und/oder einer Dodecandisäureanlage und/oder einer Hydroxylaminanlage und/oder einer mit dem Abgas einer Adipinsäureanlage und/oder einer Dodecandisäureanlage und/oder einer Hydroxylaminanlage betriebenen Salpetersäureanlage eingesetzt wird.

Das erfindungsgemäße Verfahren wird in einem Kontzwierlichem Reaktor durchgeführt, Ebenso ist die Kombination aus zwei oder mehr gleichen oder verschiedener Reaktoren möglich. Unter anderem kann die Umsetzung gemäß (iii) beispielsweise in mindestens einer Blasensäule durchgeführt werden. Die Umsetzung gemäß (iii) wird in mindestens einem kontinuierlichen Reaktor durchgeführt. Beispielsweise kann die Umsetzung gemäß (iii) in einem CSTR (continuous stirred tank reactor) oder in einer CSTR-Kaskade erfolgen. Bevorzugt ist mindestens einer der mindestens einen kontinuierlichen Reaktoren ein kontinuierlicher Rohrreaktor.

Demgemäß betrifft die vorliegende Erfindung ein Verfahren, wie oben beschrieben, das dadurch gekennzeichnet ist, dass gemäß Schritt (iü) die Gemische G(i) und G(ii) in einem kontinuierlichen Reaktor, insbesondere in einem kontinuierlichen Rohrreaktor in Kontakt gebracht werden.

In einer weiter bevorzugten Ausführungsform ist mindestens einer der erfindungsgemäß eingesetzten kontinuierlichen Rohrreaktoren ein Rohrbündelreaktor.

Bevorzugt werden die Reaktionsbedingungen in dem mindestens einen kontinuierlichen Reaktor so gewählt, dass die Umsetzung gemäß (iii) in flüssiger oder überkritischer Phase erfolgt. Weiter bevorzugt sind Reaktionsbedingungen, bei denen der gesamte Reaktorinhalt flüssig ist. Unter dem Begriff "Reaktorinhalt" werden dabei die Gemische G(i) und G(ii) verstanden, nachdem sie in den Reaktor eingebracht wurden, sowie die aus diesen Gemischen resultierende Mischung. Insbesondere bevorzugt werden die Gemische G(i) und G(ii) getrennt voneinander in den Reaktor eingeführt.

Demgemäß betrifft die vorliegende Erfindung auch ein Verfahren, wie oben beschrieben, das dadurch gekennzeichnet ist, dass der kontinuierliche Reaktor, insbesondere der kontinuierliche Rohrreaktor während der Umsetzung gemäß (iii) im wesentlichen ausschließlich mit Flüssigkeit gefüllt ist.
Ebenso betrifft die vorliegende Erfindung ein Verfahren wie oben beschrieben, das dadurch gekennzeichnet ist, dass sich der Reaktorinhalt im kontinuierlichen Reaktor, insbesondere im kontinuierlichen Rohrreaktor während der Umsetzung gemäß (iii) im Wesentlichen in überkritischer Phase befindet.

Ganz besonders bevorzugt werden die Reaktionsbedingungen so gewählt, dass das sich im Reaktor befindliche Gemisch homogen und einphasig ist.

Das Gemisch G(i) wird im allgemeinen bei Temperaturen im Bereich von 0 bis 320°C, bevorzugt im Bereich von 180 bis 300°C und besonders bevorzugt im Bereich von 200 bis 290°C in den kontinuierlichen Reaktor eingebracht, wobei die Drücke im allgemeinen im Bereich von 1 bis 500 bar, bevorzugt im Bereich von 10 bis 365 bar und besonders bevorzugt im Bereich von 25 bis 300 bar liegen.

Das Gemisch G(ii) wird im allgemeinen bei Temperaturen im Bereich von 0 bis 320°C, bevorzugt im Bereich von 180 bis 300°C und besonders bevorzugt im Bereich von 200 bis 290°C in den kontinuierlichen Reaktor eingebracht, wobei die Drücke im allgemeinen im Bereich von 5 bis 500 bar, bevorzugt im Bereich von 10 bis 365 bar und besonders bevorzugt im Bereich von 25 bis 300 bar liegen.

Im kontinuierlichen Reaktor werden die Gemische G(i) und G(ii) in Kontakt gebracht. Das Gemisch G(i) und das Gemisch (ii) beziehungsweise das reine N₂O werden in solchen Verhältnissen eingesetzt, bei denen das molare Verhältnis von Cyclopenten zu N₂O im allgemeinen im Bereich von 0,05 bis 10, bevorzugt im Bereich von 0,5 bis 7 und besonders bevorzugt im Bereich von 1 bis 5 liegt.

Ebenso ist es im Rahmen der vorliegenden Erfindung jedoch möglich, das Gemisch G(ii) und das Gemisch G(i) oder einen Teil des Gemischs G(i) zu mischen und dieses Gemisch in den Reaktor einzuführen. Erfindungsgemäß werden das Gemisch G(ii) und das Gemisch G(i) oder einen Teil des Gemischs G(i) bei Temperaturen gemischt, bei denen keine Reaktion stattfindet. Vorzugsweise erfolgt das Mischen bei einer Temperatur im Bereich von 80 bis 200°C, bevorzugt im Bereich von 90 bis 150°C, insbesondere im Bereich von 100 bis 120°C.

Die Umsetzung des im Gemisch G(i) enthaltenen Cyclopenten mit dem im Gemisch G(ii) enthaltenen N₂O in dem mindestens einen kontinuierlichen Reaktor erfolgt bei Temperaturen im Bereich von 200 bis 290°C. Die Drücke im kontinuierlichen Reaktor liegen dabei im Bereich von 10 bis 400 bar.

Die Verweilzeit des Reaktionsgemisches im kontinuierlichen Reaktor liegt im Bereich von 0,3 bis 2,5 h. Dabei ist es denkbar, die Temperatur oder den Druck oder beide im Reaktor nicht konstant zu halten, sondern in den oben angegebenen Grenzen geeignet zu variieren.

Durch das erfindungsgemäße Verfahren gemäß der oben beschriebenen Verfahrensführungen unter Verwendung der Gemische G(i) und G(ii) werden Cyclopentenumsätze erreicht, die im allgemeinen bei mindestens 10 %, bevorzugt bei mindestens 20%, insbesondere bei mindestens 30 % und weiter bevorzugt bei mindestens 50 % liegen. Die Obergrenze der Umsätze liegt im Allgemeinen bei 90%, insbesondere bei 98 %, bevorzugt bei 99 % und besonders bevorzugt bei 99,9 %.

Die Cyclopentanonselektivitäten der Umsetzung bezüglich Cyclopenten liegen hierbei im Allgemeinen im Bereich von 92 bis 99,5 %.

Demgemäß beschreibt die vorliegende Erfindung auch ein Verfahren, wie oben beschrieben, das dadurch gekennzeichnet ist, dass das eingesetzte Cyclopenten im Bereich von 10 bis 99,9 %, bezogen auf die eingesetzte Gesamtmenge an Cyclopenten, mit einer Cyclopentanonselektivität bezüglich Cyclopenten im Bereich von 92 bis 99,5 % umgesetzt wird.

Das gemäß Schritt (iii) erhaltene und Cyclopentanon enthaltende Gemisch kann im weiteren gemäß sämtlicher geeigneter Verfahren zur Gewinnung des Cyclopentanons aufgearbeitet werden. Besonders bevorzugt sind hierbei destillative Verfahren zu nennen.

In den nachfolgenden Beispielen wird die Erfindung näher erläutert.

### Beispiele

### Beispiel 1: Oxidation von Cyclopenten/Cyclopentan zu Cyclopentanon

Aus entsprechenden Vorlagebehältern werden 2000 g/h eines ca. 1:1 Gemisches Cyclopenten/Cyclopentan (davon 400 g/h frisch und 1600 g/h rückgeführtes Edukt, Cyclopenten/Cyclopentan 1:1) und 140 g/h eines flüssigen Gemisches mit 93,5 Gew.-% N₂O über einen statischen Mischer in einen Rohrreaktor (Doppelmantelrohr, gewickelt, Innendurchmesser 6 mm, Länge 85 m) gepumpt. Das Rohr wird durch Umpumpen von Wärmeträgeröl im Mantel auf 280°C thermostatisiert. Der Reaktorinnendruck wird auf 300 bar geregelt. Nach Passieren der Reaktionszone wird das Reaktionsgemisch in zwei Flashbehältern zunächst auf 16 bar und anschließend auf 5 bar entspannt, um gebildeten N₂, unumgesetztes N₂O und im N₂O enthaltene Inerte (hauptsächlich N₂ und CO₂) abzuführen.

Das flüssige Produkt wird dann in einer Kolonne mit mindestens 27 theoretischen Trennstufen bei 1 bar destilliert (T_{Sumpf}=79°C, T_{Kopf}=46°C). Als Kopfprodukt erhält man im Mittel 1600 g/h unumgesetztes Cyclopenten im Gemisch (ca. 1:1) mit Cyclopentan, das wieder in die Reaktion zurückgeführt wird. Der Sumpfaustrag, der zu ca. 50 % Cyclopentanon (Rohcyclopentanon) und ca. 50 % Cyclopentan enthält, wird in ein Produktfass überführt.

Es werden im Mittel 237 g/h Rohcyclopentanon erhalten, das in einer weiteren Destillation noch vom Cyclopentan getrennt werden muss. Die Selektivität zu Cyclopentanon beträgt 96% (bezogen auf umgesetztes Cyclopenten).

## Patentansprüche

1. Verfahren zur Herstellung von Cyclopentanon, mindestens umfassend folgende Schritte (i) bis (iii):
(i) Bereitstellen eines Cyclopenten enthaltenden Gemisches G(i);
(ii) Bereitstellen von flüssigem oder überkritischem N₂O oder eines verflüssigten oder überkritischen Gasgemisches G(ii), enthaltend mindestens 20 Vol.-% N₂O, bezogen auf das Gesamtvolumen des Gemisches G(ii);
(iii) Inkontaktbringen des Gemisches G(i) mit dem flüssigen oder überkritischen N₂O oder mit dem verflüssigten oder überkritischen Gemisch G(ii) unter Erhalt eines Gemisches G(iii), enthaltend Cyclopentanon,
**dadurch gekennzeichnet, dass** das Gemisch G(i) mindestens 25 Gew.-% und höchstens 95 Gew.-% Cyclopenten, bezogen auf das Gesamtgewicht des Gemisches G(i), enthält,
und wobei in Schritt (iii) die Gemische G(i) und G(ii) in einem kontinuierlichen Reaktor in Kontakt gebracht werden bei Temperaturen im Bereich von 200 bis 290°C und Drücken im Bereich von 10 bis 400 bar, wobei die Verweilzeit des Reaktionsgemisches im kontinuierlichen Reaktor im Bereich von 0,3 bis 2,5 h liegt.

2. Verfahren nach Anspruch 1, **dadurch gekennzeichnet, dass** das Gemisch G(i) zusätzlich zu Cyclopenten mindestens eine beim Inkontaktbringen gemäss (iii) gegenüber N₂O inerte Verbindung enthält.

3. Verfahren nach Anspruch 1 oder 2, **dadurch gekennzeichnet, dass** das Gemisch G(i) zu mindestens 99 Gew.-%, bezogen auf das Gesamtgewicht des Gemisches, Kohlenwasserstoffe enthält.

4. Verfahren nach einem der Ansprüche 1 bis 3, **dadurch gekennzeichnet, dass** das Gemisch G(i) zu mindestens 99 Gew.-% C₅- und C₆- oder C₅- und C₇- oder C₅- und C₆- und C₇-Kohlenwasserstoffe enthält.

5. Verfahren nach einem der Ansprüche 1 bis 4, **dadurch gekennzeichnet, dass** das Gemisch G(ii) höchstens 93 Vol.-% N₂O enthält.

6. Verfahren nach einem der Ansprüche 1 bis 5, **dadurch gekennzeichnet, dass** das Gemisch G(ii) höchstens 0,5 Vol-% Sauerstoff oder höchstens 0,5 Vol-% Stickoxide oder höchstens sowohl 0,5 Vol-% Sauerstoff als auch 0,5 Vol-% Stickoxide, jeweils bezogen auf das Gesamtvolumen des Gemisches G(ii), enthält.

7. Verfahren nach einem der Ansprüche 1 bis 6, **dadurch gekennzeichnet, dass** das Gasgemisch G(ii) vor dem Einsatz im Verfahren mindestens einem Reinigungsschritt unterworfen wird, in welchem der Gehalt an Sauerstoff und/oder an Stickoxiden auf höchstens 0,5 Vol.-% eingestellt wird.

8. Verfahren nach Anspruch 7, **dadurch gekennzeichnet, dass** der kontinuierliche Reaktor während der Umsetzung gemäss (iii) im Wesentlichen ausschliesslich mit Flüssigkeit gefüllt ist.

9. Verfahren nach Anspruch 7 oder 8, **dadurch gekennzeichnet, dass** sich der Reaktorinhalt im kontinuierlichen Reaktor während der Umsetzung gemäss (iii) im Wesentlichen in überkritischer Phase befindet.

## Claims

1. A process for preparing cyclopentanone, at least comprising the following steps (i) to (iii):
(i) providing a mixture G(i) comprising cyclopentene;
(ii) providing liquid or supercritical N₂O or a liquid or supercritical gas mixture G(ii) comprising at least 20% by volume of N₂O, based on the total volume of the mixture G (ii) ;
(iii) contacting the mixture G(i) with the liquid or supercritical N₂O or with the liquid or supercritical mixture G(ii) to obtain a mixture G(iii) comprising cyclopentanone,
wherein the mixture G(i) comprises at least 25% by weight and at most 95% by weight, of cyclopentene, based on the total weight of the mixture G(i),
and where, in step (iii), the mixtures G(i) and G(ii) are contacted in a continuous reactor at temperatures in the range from 200 to 290°C and pressures in the range from 10 to 400 bar, where the residence time of the reaction mixture in the continuous reactor is in the range from 0.3 to 2.5 h.

2. The process according to claim 1, wherein the mixture G(i), in addition to cyclopentene, comprises at least one compound which is inert toward N₂O in the course of the contacting of (iii).

3. The process according to claim 1 or 2, wherein the mixture G(i) comprises at least 99% by weight, based on the total weight of the mixture, of hydrocarbons.

4. The process according to any of claims 1 to 3, wherein the mixture G(i) comprises at least 99% by weight of C₅ and C₆ or C₅ and C₇ or C₅ and C₆ and C₇ hydrocarbons.

5. The process according to any of claims 1 to 4, wherein the mixture G(ii) comprises at most 93% by volume of N₂O.

6. The process according to any of claims 1 to 5, wherein the mixture G(ii) comprises at most 0.5% by volume of oxygen or at most 0.5% by volume of nitrogen oxides or at most both 0.5% by volume of oxygen and 0.5% by volume of nitrogen oxides, based in each case on the total volume of the mixture G (ii) .

7. The process according to any of claims 1 to 6, wherein the gas mixture G(ii) is subjected, before use in the process, to at least one purification step in which the content of oxygen and/or of nitrogen oxides is set to at most 0.5% by volume.

8. The process according to claim 7, wherein the continuous reactor is filled substantially exclusively with liquid during the conversion of (iii).

9. The process according to either of claims 7 and 8, wherein the reactor contents in the continuous reactor are substantially in the supercritical phase during the reaction in (iii).

## Revendications

1. Procédé pour la préparation de cyclopentanone, comprenant au moins les étapes (i) à (iii) suivantes .
(i) mise à disposition d'un mélange contenant du cyclopentène G(i) ;
(ii) mise à disposition de N₂O fluide ou supercritique ou d'un mélange gazeux liquéfié ou supercritique G(ii), contenant au moins 20% en volume de N₂O, par rapport au volume total du mélange G(ii) ;
(iii) mise en contact du mélange G(i) avec le N₂O liquide ou supercritique ou le mélange liquéfié ou supercritique G(ii) avec obtention d'un mélange G(iii), contenant de la cyclopentanone,
**caractérisé en ce que** le mélange G(i) contient au moins 25% en poids et au plus 95% en poids de cyclopentène, par rapport au poids total du mélange G(i),
et les mélanges G(i) et G(ii) étant mis en contact dans l'étape (iii) dans un réacteur continu à des températures dans la plage de 200 à 290°C et à des pressions dans la plage de 10 à 400 bars, la durée de séjour du mélange réactionnel dans le réacteur continu étant situé dans la plage de 0,3 à 2,5 h.

2. Procédé selon la revendication 1, **caractérisé en ce que** le mélange G(i) contient, en plus du cyclopentène, au moins un composé inerte par rapport à N₂O lors de la mise en contact selon l'étape (iii).

3. Procédé selon la revendication 1 ou 2, **caractérisé en ce que** le mélange G(i) contient des hydrocarbures à raison d'au moins 99% en poids par rapport au poids total du mélange.

4. Procédé selon l'une quelconque des revendications 1 à 3, **caractérisé en ce que** le mélange G (i) contient des hydrocarbures en C₅ et en C₆ ou en C₅ et en C₇ ou en C₅ et C₆ et en C₇ à raison d'au moins 99% en poids.

5. Procédé selon l'une quelconque des revendications 1 à 4, **caractérisé en ce que** le mélange G(ii) contient au plus 93% en volume de N₂O.

6. Procédé selon l'une quelconque des revendications 1 à 5, **caractérisé en ce que** le mélange G(ii) contient au plus 0,5% en volume d'oxygène ou au plus 0,5% en volume d'oxydes d'azote ou au plus tant 0,5% en volume d'oxygène que 0,5% en volume d'oxydes d'azote, à chaque fois par rapport au volume total du mélange G (ii) .

7. Procédé selon l'une quelconque des revendications 1 à 6, **caractérisé en ce que** le mélange gazeux G(ii) est soumis, avant l'utilisation dans le procédé, à au moins une étape de purification dans laquelle la teneur en oxygène et/ou en oxydes d'azote est réglée à au plus 0,5% en volume.

8. Procédé selon la revendication 7, **caractérisé en ce que** le réacteur continu est rempli de manière sensiblement exclusive par un liquide pendant la transformation selon l'étape (iii).

9. Procédé selon la revendication 7 ou 8, **caractérisé en ce que** le contenu dans le réacteur continu se trouve dans une phase sensiblement surcritique pendant la transformation selon l'étape (iii).
